# EUROPÄISCHE PATENTANMELDUNG

(11) **EP 0 806 250 A2**
(43) Veröffentlichungstag der Anmeldung: **12.11.1997**
(21) Anmeldenummer: 97107572.6
(22) Anmeldetag: 07.05.1997
(51) Int. Cl.: B05D 7/24, G01N 33/543

(54) **Mit Aminogruppen beschichtete Oberfläche**

(30) Priorität: 10.05.1996 DE 19618926
(71) Anmelder: BOEHRINGER MANNHEIM GMBH, 68305 Mannheim (DE)
(72) Erfinder: Sluka, Peter, Dr., 82362 Weilheim (DE); Beyer, Dierk, Dr., 55130 Mainz (DE); Ringsdorf, Helmut, Prof. Dr., 55124 Mainz-Gonsenheim (DE); Knoll, Wolfgang, Prof. Dr., 55124 Mainz (DE)
(74) Vertreter: Böhm, Brigitte, Dipl.-Chem. Dr.

(57) **Zusammenfassung**

Eine mit Aminogruppen beschichtete Oberfläche wird durch Aufbringen eines polymerisierbaren Amins mittels gepulsten Plasmas auf eine Oberfläche hergestellt. Die dadurch erhaltenen beschichteten Oberflächen weisen eine hohe Dichte an Aminogruppen auf, so daß eine spezifische Bindephase durch kovalentes Binden eines Partners eines spezifischen Bindepaares an die mit Aminogruppen beschichteten Oberflächen erhalten werden kann.

## Beschreibung

Die Erfindung betrifft ein Verfahren zur Herstellung einer mit Aminogruppen beschichteten Oberfläche sowie eine Oberfläche mit hoher Aminogruppendichte. Die Erfindung betrifft weiterhin ein Verfahren zur Herstellung einer spezifischen Bindephase unter Verwendung der mit Aminogruppen beschichteten Oberfläche, eine spezifische Bindephase mit verbesserten Eigenschaften sowie deren Verwendung in Immunoassays und in der Medizintechnik.

Die Wechselwirkungen von Proteinen mit Polymeroberflächen spielen eine wichtige Rolle in der Diagnostik. Polymeroberflächen werden beispielsweise als Festphasen für Immunoassays oder als Implantate in der Medizintechnik verwendet. Im Fall von Immunoassays werden üblicherweise gegen den zu bestimmenden Analyten gerichtete spezifische Antikörper adsorptiv an die Oberfläche gebunden. Probleme bereitet dabei allerdings das oftmals unterschiedliche Bindeverhalten von Proteinen an Oberflächen, welches Ausbluten des Antikörpers oder Verdrängungsreaktionen zur Folge haben kann. Diese Probleme können zum Teil durch universelle Bindesysteme, wie beipielsweise das Streptavidin/Biotin-System, verringert werden.

Ein weiteres Problem bei Immunoassays ist das Auftreten unspezifischer Bindung von weiteren Probenbestandteilen, wie Serum- oder Plasmaproteinen, an die Oberfläche, was zu einer Verringerung der Sensitivität und Spezifität des Nachweisverfahrens führt. Solche unspezifischen Wechselwirkungen können zu einer erniedrigten Bindung des Analyten oder zu falsch-positiven Resultaten führen. Dieses Problem der unspezifischen Bindung tritt bei allen Nachweisverfahren auf, in denen in der Probe neben dem Analyten noch weitere Bestandteile vorliegen, die an die Oberfläche adsorbieren können. Beispiele für solche Proben sind biologische Proben, wie etwa Blut, Blutbestandteile, Serum etc.

In der Vergangenheit wurden verschiedene Möglichkeiten beschrieben, Polymeroberflächen im Hinblick auf eine Verringerung unspezifischer Proteinbindung zu modifizieren. Allgemein wurde dabei versucht, sich die adsorptionsvermindernde Wirkung von immobilisierten Polyethylenglykolen (PEG) zunutze zu machen. N. Desai und J. Hubbell, J. Biomed. Mat. Res., Vol. 25 (1991), 829-843 beschreiben eine naßchemische Modifizierung von Polyethylenterephthalat-Oberflächen durch Aminolyse und anschließende Bindung von Cyanurchlorid-aktivierten Polyethylenglykolen an die Oberfläche. Allerdings konnte erst bei einem Polyethylenglykol-Molgewicht von mindestens 18.500 eine deutliche Verringerung der Proteinbindung am Beispiel von Fibrinogen gezeigt werden. Nachteilig ist weiterhin, daß die Aminolyse als Verfahren kaum steuerbar und somit nur schwer reproduzierbar ist.

E. Kiss et al., Progr. Colloid & Polymer Sci., 74 (1987), 113-119 beschreiben eine Modifizierung von Oberflächen durch Aufpfropfen von Polyethylenglykol auf Polyethylen in wäßrigem Medium. Dazu wurde Polyethylenglykolaldehyd mittels reduktiver Aminierung an Aminogruppen von Polyethylenimin gekoppelt, das auf die Polyethylenoberfläche adsorbiert war. Auch dieses Verfahren ist schlecht reproduzierbar und die Modifizierung der Oberfläche erfolgt lediglich statistisch.

E. Uchida et al., Langmuir, 10 (1994), 481-485 beschreiben ein Verfahren zur Modifizierung von Polymeroberflächen durch Aufbringen von acrylderivatisierten Polyethylenglykolen mittels photoinduzierter Pfropfung auf Polyethylenterephthalat-Oberflächen. WO92/07006 beschreibt die Modifizierung von geladenen Oberflächen in wäßrigem Medium mit polyethylengekoppelten Polyethyleniminen. Die Bindung des Polymers an die Oberfläche kommt dabei durch rein ionische Wechselwirkungen zustande, so daß die beschichtete Oberfläche empfindlich gegenüber pH-Wertschwankungen ist.

Von Ratner et al., J. Biomed. Mat. Res., Vol. 26 (1992), 415-439 wurde der Versuch unternommen, Kunststoffoberflächen durch continous wave (cw)-Plasmabehandlung in Gegenwart von Tetraethylenglykoldimethylether zu modifizieren. Diese modifizierten Oberflächen weisen eine schlecht reproduzierbare Herstellbarkeit auf sowie eine geringe Stabilität gegenüber Wasser, was zu teilweisem Ablösen der Schicht führt. Da die Schichten zudem sehr dünn sind, ist eine weitere Modifizierung praktisch nicht möglich.

Die Modifizierung der Oberflächen erfolgt bei den im Stand der Technik bekannten Verfahren lediglich statistisch und ist schlecht reproduzierbar. Sowohl die Modifizierung von Oberflächen in wäßriger Umgebung als auch durch Behandlung mit continuous wave Plasma (cw-Plasma) führt zu einer ungleichmäßigen statistischen Verteilung der Aminogruppen auf der Oberfläche. Deshalb müssen relativ langkettige Polyethylenglykolderivate immobilisiert werden, um eine Verringerung von unspezifischen Bindungen zu erreichen. Das Aufbringen von spezifischen Bindungsstellen für Analyten, wie sie für die Verwendung der modifizierten Oberflächen in Immunoassays erforderlich ist, gleichzeitig mit PEG ist bei diesen Ansätzen praktisch nicht möglich. Weiterhin ist es bei den bisher bekannten modifizierten Oberflächen, die mittels cw-Plasma beschichtet wurden, notwendig, nachfolgende chemische Modifizierungen unter Verwendung von organischen Lösungsmitteln auszuführen. Lösungsmittelempfindliche Polymere, wie z.B. Polystyrol, die in der Diagnostik eine große Rolle spielen, können auf diese Art nicht modifiziert werden.

Es war daher Aufgabe der Erfindung, ein Verfahren zur Herstellung einer mit derivatisierbaren Aminogruppen beschichteten Oberfläche mit verbesserten Eigenschaften herzustellen, mittels derer eine spezifische Bindephase erhalten werden kann, die eine hohe Sensitivität und Spezifität aufweist.

Die Aufgabe wird erfindungsgemäß gelöst durch ein Verfahren zur Herstellung einer mit Aminogruppen beschichteten Oberfläche, worin ein polymerisierbares Amin mittels gepulsten Plasmas auf eine Oberfläche aufgebracht wird.

Die Verwendung eines gepulsten Plasmas, wie es beispielsweise bei V. Panchalingam et al., J. Biomater. Sci. Polymer Edn., Vol. 5, Nr. 1/2 (1993), 131-145 beschrieben ist, aus einem polymerisierbaren Amin, führt überraschenderweise zu einer Oberfläche, die ca. 80 % mehr Stickstoff in Form von Aminogruppen enthält als herkömmliche, z.B. mittels konventioneller continuous wave Plasmatechnik hergestellte Oberflächen. Die gebildeten Oberflächen weisen eine gleichmäßige Aminbeschichtung auf und sind mit guter Reproduzierbarkeit herstellbar.

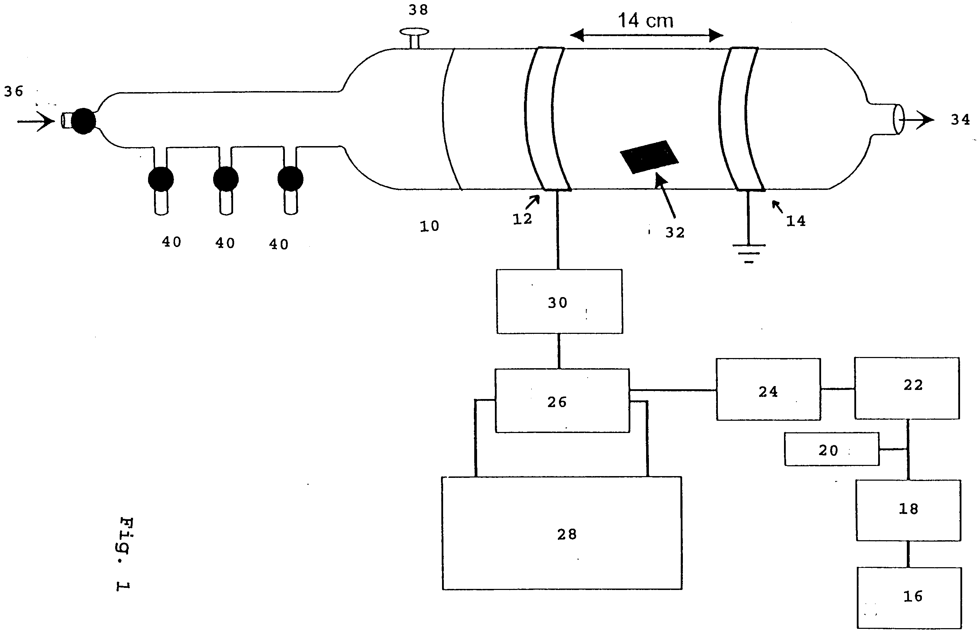

Das gepulste Plasma wird beispielsweise in einem Plasmareaktor gemäß Figur 1 erzeugt. In einem evakuierbaren Behälter (10) sind zwei Elektroden (12) und (14) angebracht. Die Elektrode (12) ist an eine Apparatur zur Erzeugung von Radiofrequenzpulsen angeschlossen, die aus einem Pulsgenerator (16), einem Radiofrequenzgenerator (18), einem Zähler (20), einem Radiofrequenzverstärker (22), einem Wattmeter (24), einem Zweirichtungskoppler (26), einem Oszilloskop (28) sowie einem Anpassungsnetzwerk (30) besteht. Radiofrequenzpulse werden mittels Pulsgenerator (16) und Radiofrequenzgenerator (18) erzeugt und durch den Radiofrequenzverstärker (22) bis zur gewünschten Leistung verstärkt. Der Zähler (20) sowie das Wattmeter (24) dienen dazu, die Pulsdauern, Pulsfrequenzen sowie die Pulsleistungen zu beobachten. Die Radiofrequenzpulse werden zu einem Zweirichtungskoppler (26) geleitet. Von diesem wird Leistung zu einem Oszilloskop (28) geleitet, das die Leistung zum Zweirichtungskoppler (26) reflektiert. Die Radiofrequenzpulse werden dann über ein Anpassungsnetzwerk (30) an die Elektrode (12) angelegt.

Die zu beschichtende Oberfläche (32) wird in den evakuierbaren Behälter (10) zwischen die Elektroden (12) und (14) gebracht.

Der Reaktor wird durch eine Vakuumpumpe (34) evakuiert und polymerisierbares Amin wird durch einen Einlaß (36) zugeführt. Der Druck wird durch ein Druckmeßgerät (38) überwacht. An der Vorrichtung sind zudem noch weitere Einlaßöffnungen (40) vorgesehen, durch die weitere Substanzen, z.B. Argon, eingeleitet werden können. Ein gepulstes Plasma wird dadurch erzeugt, daß die zur Plasmaerzeugung erforderliche Leistung abwechselnd ein- und ausgeschaltet wird, wodurch ein Plasma aus dem polymerisierbaren Amin gebildet wird.

Zur Erzielung einer gleichmäßigen reproduzierbaren Beschichtung der Oberfläche mit einer hohen Aminogruppendichte wird ein gepulstes Plasma verwendet, das Pulsdauern von 0,5 bis 1000 ms und eine Pulsfrequenz von 0,5 bis 1000 Hz aufweist. Je nach gewünschter Beschichtungsdichte beträgt die Behandlungszeit zwischen 1 Minute und 2 Stunden. Bevorzugt weist das gepulste Plasma Anzeiten von 1 bis 100 ms und besonders bevorzugt von 5 bis 50 ms auf. Die Auszeiten liegen zwischen 0,5 und 1000 ms, bevorzugt zwischen 1 und 200 ms und besonders bevorzugt zwischen 5 und 100 ms. Die zur Plasmaerzeugung eingesetzte Leistung beträgt bevorzugt zwischen 200 und 500 Watt und besonders bevorzugt zwischen 50 und 300 Watt. Die geeignete Radiofrequenz ist abhängig vom Reaktortyp und beträgt für den in Figur 1 dargestellten Reaktor etwa 13,56 MHz.

Das polymerisierbare Amin kann mittels gepulsten Plasmas in einem Arbeitsschritt auf die Oberfläche aufgebracht werden. Es ist allerdings bevorzugt, zunächst eine Reinigung der Substrate mittels gepulsten Argonplasmas durchzuführen und anschließend das polymerisierbare Amin in zwei Schritten auf die Oberfläche aufzubringen, nämlich einem Grundierungsschritt und einem Beschichtungsschritt. Die Grundierung erfolgt dabei üblicherweise mit kürzeren Auszeiten.

Als polymerisierbares Amin kann ein primäres oder sekundäres Amin verwendet werden, welches zusätzlich zu der Aminogruppe eine polymerisierbare Gruppe aufweist. Bei der polymerisierbaren Gruppe handelt es sich bevorzugt um eine ungesättigte Gruppe, wie beispielsweise eine C=C-Doppelbindung. Das polymerisierbare Amin bzw. das angelegte Vakuum müssen so ausgewählt werden, daß der Dampfdruck des polymerisierbaren Amins ausreicht, daß zumindest ein Teil des Amins bei den angelegten Vakuumbedingungen in der Gasphase vorliegt. Bevorzugt werden deshalb leicht flüchtige polymerisierbare Amine mit hohem Dampfdruck verwendet. Beispiele für erfindungsgemäß geeignete Amine sind primäre und sekundäre C₂-C₈-Alkenyle sowie Styrolderivate, die eine primäre oder sekundäre Aminogruppe aufweisen. Besonders bevorzugt wird als polymerisierbares Amin Allylamin verwendet.

Erfindungsgemäß besteht die Oberfläche aus einem Kunststoff. Insbesondere sind spritzgußfähige Kunststoffe, wie Polycarbonat, Polyethylen, Polypropylen usw., geeignet. Besonders bevorzugt besteht die Oberfläche aus Polystyrol.

Ein weiterer Gegenstand der Erfindung ist eine nach dem obenbeschriebenen Verfahren erhältliche mit Aminogruppen beschichtete Oberfläche. Eine solche Oberfläche weist bevorzugt eine Dichte der die Aminogruppen enthaltenden Moleküle auf der Oberfläche von mindestens 80 % der vollständigen Belegung, besonders bevorzugt von mindestens 95 % der vollständigen Belegung auf. Unter vollständiger Belegung ist dabei die dichteste, theoretisch im Hinblick auf die Molekülgröße mögliche Beschichtung der Oberfläche mit einer monomolekularen Schicht aus Aminmolekülen zu verstehen. Die Beschichtungsdichte kann beispielsweise mittels ESCA-Spektroskopie bestimmt werden.

Die hohe Dichte und gleichmäßige Verteilung der Aminogruppen führt überraschenderweise dazu, daß die erfindungsgemäßen Oberflächen reproduzierbar modifiziert werden können. Die Modifizierung erfolgt geeigneterweise durch Derivatisierung der primären oder sekundären Aminogruppen. Dadurch können an die Oberfläche Moleküle mit wünschenswerten funktionellen Gruppen oder Eigenschaften gebunden werden.

Beispielsweise ermöglicht die mit dem gepulsten Plasma erzielte reproduzierbare hohe Amindichte auf der Oberfläche die anschließende kovalente Bindung eines Partners eines spezifischen Bindepaares an die mit Aminogruppen beschichtete Oberfläche.

Deshalb ist ein Verfahren zur Herstellung einer spezifischen Bindephase ein weiterer Gegenstand der Erfindung. Dabei wird ein Partner eines spezifischen Bindepaares kovalent an eine gleichmäßig und mit hoher Dichte mit Aminogruppen beschichtete Oberfläche gebunden.

Spezifische Bindepaare sind dem Fachmann bekannt und umfassen beispielsweise das Streptavidin-Biotin-System, aber auch Antikörper-Antigen- bzw. Hapten-Paare. Bevorzugt wird Biotin oder ein Biotinanalogon wie Iminobiotin oder Desthiobiotin verwendet. Unter Biotinanalogon ist dabei jedes mit Streptavidin bindefähige Molekül zu verstehen.

Die Oberfläche kann somit gezielt mit spezifischen Bindestellen ausgestattet werden, die z.B. direkt mit einem Analyten oder mit einem spezifischen Bindepartner bindefähig sind. Andererseits kann die Oberfläche auch mit Molekülen, wie z.B. Polyethylenglykolen, beschichtet werden, die unspezifische Bindungen von Probenbestandteilen an die Oberfläche verringern, um Oberflächen zu bilden, an die unerwünschte Adsorption oder Bindung praktisch nicht stattfindet. Solche Oberflächen finden beispielsweise als Implantate in der Medizintechnik Anwendung.

Bevorzugt wird zusätzlich zu dem Partner eines spezifischen Bindepaares Polyethylenglykol oder ein Polyethylenglykolderivat an die mit Aminogruppen beschichtete Oberfläche gebunden, um eine spezifische Bindephase zu erhalten, bei der unspezifische Wechselwirkungen weiter reduziert sind. Das Polyethylenglykol weist dabei eine mittlere Kettenlänge von 3 bis 200 Monomereinheiten, bevorzugt von 3 bis 50 Monomereinheiten und besonders bevorzugt von 3 bis 10 Monomereinheiten auf. Das Polyethylenglykol kann hydroxy- oder alkylterminiert sein.

Bevorzugt werden Polyglykol und der Partner eines spezifischen Bindepaares zur Bindung an die Oberfläche in einem Molverhältnis von 0 : 100 bis 99 : 1 eingesetzt, bevorzugt in einem Molverhältnis von 70 : 30 bis 99 : 1 und besonders bevorzugt von 80 : 20 bis 99 : 1. Überraschenderweise konnte z.B. die Bindung von Fibrinogen bereits durch die Verwendung von kurzkettigen Polyethylenglykolderivaten vermieden werden. Der gleichzeitige Einbau von Partnern eines spezifischen Bindepaares, wie beispielsweise Biotin und PEG, ermöglicht die spezifische Bindung von z.B. Streptavidin an die Oberflächen bei gleichzeitiger Minimierung der unspezifischen Bindung anderer Probenbestandteile, wie z.B. Fibrinogen.

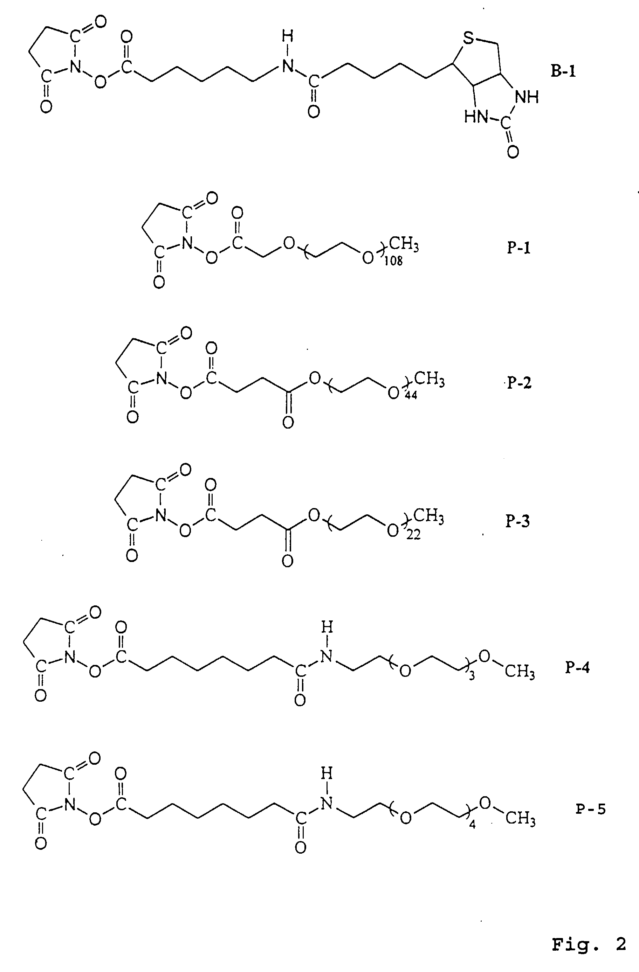

Die hohe Dichte und gleichmäßige Verteilung der Aminogruppen auf der Oberfläche führt überraschenderweise dazu, daß die Bindung des Partners eines spezifischen Bindepaares und gegebenenfalls des Polyethylenglykols an die mit Aminogruppen beschichtete Oberfläche in wäßriger Lösung durchgeführt werden kann. Beispielsweise können der Partner eines spezifischen Bindepaares und/oder das Polyethylenglykol als Aktivesterderivat eingesetzt werden. Unter Aktivesterderivat ist dabei ein Derivat des Partners eines spezifischen Bindepaares oder des Polyethylenglykols zu verstehen, das eine aktivierte Estergruppe umfaßt, die mit einer Aminogruppe im wäßrigen Milieu reagieren kann. Beispiele solcher Aktivesterderivate sind in Figur 2 abgebildet.

Ein weiterer Gegenstand der Erfindung ist eine spezifische Bindephase, wie sie nach einem der oben beschriebenen Verfahren erhältlich ist. Eine solche Bindephase ist dadurch gekennzeichnet, daß der Partner eines spezifischen Bindepaares und gegebenenfalls Polyethylenglykol mit den auf der Oberfläche aufgebrachten Aminogruppen kovalent verknüpft ist. Bevorzugt ist die Oberfläche zu mehr als 80 % und besonders bevorzugt zu mehr als 95 % mit dem aufgebrachten Amin beschichtet.

Ein weiterer Gegenstand der Erfindung ist die Verwendung einer solchen spezifischen Bindephase als Festphase in einem Immunoassay. Weiterhin ist eine solche spezifische Bindephase auch zur Verwendung in der Medizintechnik, beispielsweise als Implantat, geeignet.

Die Erfindung wird durch die beigefügten Figuren und Beispiele näher erläutert.
Figur 1 zeigt eine Vorrichtung zur Aufbringung eines polymerisierbaren Amins mittels gepulsten Plasmas auf eine Oberfläche und
Figur 2 zeigt bevorzugte Beispiele von erfindungsgemäß verwendeten Aktivesterderivaten.

### Beispiel 1

### Herstellung einer Oberflächenbeschichtung durch Aufpolymerisation von Allylamin mittels gepulsten Plasmas

In der in Figur 1 dargestellten Vorrichtung erfolgte die Abscheidung von Allylamin mittels gepulsten Plasmas auf eine Oberfläche. Als Substrate wurden Polystyrol-Spritzgußteile (2 cm x 1 cm x 0,1 cm) verwendet, die zuvor in Ethanol/Wasser (1 : 2, v/v) gereinigt wurden. Die Plasmabeschichtung wurde in drei Schritten unter folgenden Bedingungen ausgeführt:
1) Zunächst wurden die Substrate mittels Argonplasma gereinigt. Dazu wurden die Polystyroloberflächen einem gepulsten Argonplasma mit Einzeiten von 10 ms, Auszeiten von 100 ms und einer Leistung von 100 Watt 10 Minuten lang ausgesetzt.
2) Nach der Reinigung wurde auf die Polystyroloberfläche eine Allylamin-"Grundierung" aufgebracht. Dies geschah mittels gepulsten Plasmas bei Einzeiten von 3 ms, Auszeiten von 5 ms und einer Leistung von 200 Watt. Der Grundierungsschritt wurde 4 Minuten lang ausgeführt.
3) Anschließend wurde eine Allylamin-"Oberflächenschicht" aufgebracht. Die Plasmabedingungen hierfür waren 3 ms Einzeit, 45 ms Auszeit und 200 Watt Leistung. Die Beschichtung wurde 10 Minuten lang ausgeführt.

### Beispiel 2

### Derivatisierung der mit Allylamin beschichteten Oberflächen

Die im gepulsten Plasma erzeugten allylaminbeschichteten Oberflächen wurden in wäßriger Lösung bei pH 8 mit den in Figur 2 dargestellten Verbindungen umgesetzt. Hierzu wurden die mit Allylamin beschichteten Oberflächen 6 Stunden in einer mit Phosphatpuffer auf pH = 8 gepufferten 10⁻³ M wäßrigen Lösung der als Aktivester vorliegenden Polyethylenglykole P1 bis P5 und des Aktivesters von Biotin B1 in den molaren Verhältnissen von 0 : 1, 1 : 1, 7 : 3 und 9 : 1 umgesetzt. Die Verbindung B-1 wurde vor dem Ansetzen der Mischung in wenig DMF gelöst und dann im entsprechenden Verhältnis der wäßrigen Lösung der Polyethylenglykole P1 bis P5 zugesetzt.

### Beispiel 3

### Analytische Charakterisierung der beschichteten Oberflächen mittels Elektronenspektroskopie für chemische Anwendungen (ESCA)

Die elementare Zusammensetzung von Oberflächenschichten kann mittels ESCA-Spektroskopie quantitativ bestimmt werden. Die mit Allylamin beschichteten Oberflächen wurden vor und nach der chemischen Derivatisierung untersucht. Bei Lagerung der frisch hergestellten, mit Aminogruppen beschichteten Oberflächen unter Wasser zeigte sich eine Änderung der Oberflächenzusammensetzung, die auf die Reaktion von Wasser mit beim Beschichtungsvorgang entstandenen reaktiven Spezies zurückzuführen ist. Die quantitative Auswertung der ESCA-Spektren nach Elementarzusammensetzung für die verschiedenen Präparate ist in folgender Tabelle zusammengestellt:

**Tabelle 1**

| **Modifikation** | **C1s [%]** | **O1s [%]** | **N1s [%]** | **S2p [%]** |
|---|---|---|---|---|
| Monomeres (berechnet) | 75,0 | -- | 25,0 | -- |
| Allylamin (cw) | 77,7 | 6,8 | 15,6 | -- |
| Allylamin (cw) nach Puffer pH=8 für 12 h | 75,4 | 11,5 | 13,1 | |
| Allylamin ("gepulst") | 71,0 | 5,2 | 23,8 | -- |
| Allylamin ("gepulst") 12 h in Puffer pH = 8 gelagert | 70,8 | 10,8 | 18,4 | -- |
| Allylamin ("gepulst") nach Reaktion mit **B-1** in Puffer | 71,4 | 10,0 | 17,5 | 1,1 |
| **P-2 : B-1** 1 : 1 | 65,9 | 21,3 | 12,1 | 0,65 |
| **P-2 : B-1** 7 : 3 | 64,5 | 23,3 | 11,9 | 0,36 |
| **P-2 : B-1** 9 : 1 | 66,7 | 24,9 | 8,1 | 0,22 |
| **P-3 : B-1** 1 : 1 | 68,4 | 17,0 | 14,1 | 0,58 |
| **P-3 : B-1** 7 : 3 | 66,8 | 22,9 | 9,9 | 0,35 |
| **P-3 : B-1** 9 : 1 | 64,3 | 25,2 | 10,4 | 0,11 |
| **P-4 : B-1** 1 : 1 | 72,5 | 11,4 | 15,5 | 0,62 |
| **P-3 : B-1** 7 : 3 | 72,8 | 12,3 | 14,5 | 0,39 |
| **P-4 : B-1** 9 : 1 | 71,4 | 13,4 | 14,9 | 0,20 |
| **P-5 : B-1** 1 : 1 | 71,3 | 11,4 | 16,6 | 0,68 |
| **P-5 : B-1** 7 : 3 | 71,9 | 11,9 | 15,8 | 0,44 |
| **P-5 : B-1** 9 : 1 | 71,5 | 13,1 | 15,3 | (0,10) |

Aus Tabelle 1 ist zu ersehen, daß mittels gepulsten Allylamin-Plasmas eine Beschichtungdichte von 23,8 (N1s, gepulstes Plasma): 25,0 (N1s, monomolekulare Schicht von Allylamin), also von 95,2 % der vollständigen Belegung erzielt wurde.

### Beispiel 4

### Proteinadsorption an die spezifischen Bindephasen

Es wurde die Bindefähigkeit der spezifischen Bindephasen gegenüber Streptavidin (spezifische Bindung) und gegenüber Fibrinogen (unspezifische Bindung) untersucht. Zum Nachweis der Fibrinogenbindung wurde ein fluoreszenzmarkiertes Fibrinogen verwendet (Rinderfibrinogen, Firma FLUKA mit Resorufin-OSu umgesetzt, Beladung 1:1). Die Streptavidinbindung wurde mit fluoreszierenden und biotinylierten Latexpartikeln (Firma Mol.-Probes L-5221, 100 nm, Farbstoff: Yellow-Green) nachgewiesen. Die Biotinylierung der Partikeloberfläche wurde durch kovalente Kopplung mit biotinyliertem Rinderserumalbumin (RSA) erreicht.

### a) Bindung von Fibrinogen

Das Konjugat wurde in einer Konzentration von 1 mg/ml in 100 mM Kaliumphosphatpuffer pH 7,4 gelöst. Zur Kontrolle wurde diese Lösung 2 Stunden auf der unbehandelten Oberfläche inkubiert. Die Lösung wurde auf die spezifischen Bindephasen aufgebracht, 2 Stunden inkubiert und anschließend mit reiner Pufferlösung abgespült. Die spezifischen Bindephasen wurden anschließend mittels Fluoreszenzmikroskop betrachtet und die Fluoreszenz der Oberfläche mittels CCD-Kamera und Bildauswertung (Optimetric, Fa. Stemer, München) als mittlere Grauwerte (mean grey) eines definierten Bildausschnitts quantifiziert.

### b) Bindung von Streptavidin

Streptavidin wurde in einer Konzentration von 0,5 mg/ml in 100 mM Kaliumphosphatpuffer, pH 7,4 gelöst. Die spezifische Bindephase wurde 2 Stunden mit der Lösung inkubiert und anschließend gewaschen. Auf die spezifische Bindephase wurde dann die Latexlösung (0,5 %ig) aufgebracht, 1 Stunde inkubiert und anschließend gewaschen. Die Fluoreszenz wurde wie unter Beispiel 4a) betrachtet und ausgewertet.

Die Ergebnisse der Proteinbindung sind in Tabelle 2 zusammengefaßt:

**Tabelle 2**

| **Oberfläche** | **Fibrinogen (mean grey) 515/560 nm** | **Streptavidin (mean grey) 450/500 nm** |
|---|---|---|
| Untergrund/Leerwert | 72 | 72 |
| Polystyrol unbehandelt | 190 | 205 |
| P-1 : B-1 7:3 | 87 | 162 |
| P-1 : B-1 9:1 | 87 | 166 |
| P-2 : B-1 7:3 | 88 | 139 |
| P-2 : B-1 9:1 | 85 | 137 |
| P-4 : B-1 7:3 | 86 | 190 |
| P-4 : B-1 9:1 | 86 | 182 |

## Patentansprüche

1. Verfahren zur Herstellung einer mit Aminogruppen beschichteten Oberfläche,
**dadurch gekennzeichnet,**
daß ein polymerisierbares Amin mittels gepulsten Plasmas auf eine Oberfläche aufgebracht wird.

2. Verfahren nach Anspruch 1,
**dadurch gekennzeichnet,**
daß man mit Pulsdauern von 0,5 - 1000 ms und einer Pulsfrequenz von 0,5 - 1000 Hz arbeitet.

3. Mit Aminogruppen beschichtete Oberfläche, erhältlich nach einem Verfahren gemäß einem der Ansprüche 1 oder 2.

4. Mit Aminogruppen beschichtete Oberfläche nach Anspruch 3,
**dadurch gekennzeichnet,**
daß die Dichte der die Aminogruppen enthaltenden Moleküle auf der Oberfläche mindestens 80 % der vollständigen Belegung beträgt.

5. Verfahren zur Herstellung einer spezifischen Bindephase,
**dadurch gekennzeichnet,**
daß ein Partner eines spezifischen Bindepaares kovalent an eine mit Aminogruppen beschichtete Oberfläche nach einem der Ansprüche 3 oder 4 gebunden wird.

6. Verfahren nach Anspruch 5,
**dadurch gekennzeichnet,**
daß zusätzlich Polyethylenglykol oder ein Polyethylenglykolderivat an die mit Aminogruppen beschichtete Oberfläche gebunden wird.

7. Verfahren nach einem der Ansprüche 5 oder 6,
**dadurch gekennzeichnet,**
daß Polyethylenglykol und der Partner eines spezifischen Bindepaares zur Bindung an die Oberfläche in einem Mol-Verhältnis von 0 : 100 bis 99 : 1 eingesetzt werden.

8. Spezifische Bindephase, erhältlich nach einem Verfahren gemäß einem der Ansprüche 5 bis 7.

9. Verwendung einer spezifischen Bindephase nach Anspruch 8 als Festphase in einem Immunoassay.

10. Verwendung einer spezifischen Bindephase nach Anspruch 8 in der Medizintechnik.
